# EUROPÄISCHE PATENTSCHRIFT

(11) **EP 2 142 470 B1**
(45) Veröffentlichungstag und Bekanntmachung des Hinweises auf die Patenterteilung: **21.06.2017**
(21) Anmeldenummer: 08735315.7
(22) Anmeldetag: 14.04.2008
(51) Int. Cl.: C01B 7/04, C01B 7/07, C07C 263/10, B01D 53/00, B01D 53/04, B01D 3/14

(54) **KONDENSATIONS-ADSORPTIONSPROZESS ZUR ENTFERNUNG ORGANISCHER KOMPONENTEN AUS EINEM CHLORWASSERSTOFF ENTHALTENDEN GASSTROM**
CONDENSATION-ADSORPTION PROCESS FOR REMOVING ORGANIC COMPONENTS FROM A GASEOUS STREAM CONTAINING HYDROGEN CHLORIDE
PROCÉDÉ D'ÉLIMINATION PAR CONDENSATION-ADSORPTION DE COMPOSANTS ORGANIQUES D'UN FLUX DE GAZ CONTENANT DU CHLORURE D'HYDROGÈNE

(30) Priorität: 26.04.2007 DE 102007020144
(43) Veröffentlichungstag der Anmeldung: 13.01.2010
(73) Patentinhaber: Covestro Deutschland AG, 51373 Leverkusen (DE)
(72) Erfinder: BRETTSCHNEIDER, Ole, 10711 Berlin (DE); WERNER, Knud, 47800 Krefeld (DE)
(74) Vertreter: Levpat
(86) Internationale Anmeldenummer: PCT/EP2008/003110
(87) Internationale Veröffentlichungsnummer: WO 2008/131873

(56) Entgegenhaltungen:
- EP-A- 0 233 773
- WO-A-2006/038705
- WO-A-2006/137583
- DE-A1- 19 534 448

## Beschreibung

Die Erfindung betrifft ein Verfahren zur Aufarbeitung von Chlorwasserstoff enthaltenden Gasströmen, die mit organischen Verbindungen verunreinigt sind, mittels einer Kombination von Kondensation und Adsorption.

Speziell betrifft die Erfindung die Reinigung von Chlorwasserstoff enthaltenden Prozessgasen der Isocyanatherstellung.

Die adsorptive Abscheidung vor allem organischer Komponenten aus Gasströmen findet häufige Anwendung in der Prozessindustrie.

Bei der Regeneration wird das Adsorbens üblicherweise erhitzt und mit einem Regenerationsgasstrom in Kontakt gebracht. Hierdurch lösen sich die adsorbierten Komponenten in den Regenerationsgasstrom und das Adsorbens wird entladen.

Aus der EP 233 773 B1 ist ein Verfahren zur katalytischen Oxidation von HCl-Gas bekannt geworden, bei dem ein mit organischen Verunreinigungen wie Benzol, Chlorbenzol und ähnlichem verunreinigtes HCl-Gas für die Verwendung in einem Deacon-Verfahren (katalytische HCl-Oxidation mittels Sauerstoff) vorgereinigt wird.

Hierzu wird Aktivkohle als Adsorber verwendet, die nach Gebrauch regeneriert wird. Es wird weiter vorgeschlagen, den Adsorber bei höheren Temperaturen oder unter vermindertem Druck und gegebenenfalls unter Verwendung eines Inertgases zu regenerieren.

Näheres über die Ausgestaltung der HCl-Reinigung ist der EP 233 773 B1 nicht zu entnehmen.

Abhängig von der Organikfracht des zu reinigenden Gasstromes und dem Dampfdruck der abzuscheidenen Organikkomponenten kann der Einsatz einer dem Adsorptionsprozess vorgelagerten Kondensation bei tiefen Temperaturen wirtschaftlich vorteilhaft sein. In diesem Fall wird in der Regel der Hauptteil der Organikfracht in einem Kondensationssystem abgeschieden, während die Nachreinigung bis auf die geforderten Reinheiten mit Hilfe eines Adsorptionsschrittes erfolgt.

Die oben beschriebene Kombination aus Kondensation und Adsorption ist Stand der Technik und beispielsweise in der US-A-5740682 beschrieben. Hierbei werden beispielsweise Kohlenwasserstoffe aus Luft entfernt.

Die oben beschriebene Kombination aus Kondensation bei tiefen Temperaturen und nachfolgender Adsorption hat bei der gegebenen Problemstellung der Entfernung organischer Komponenten aus HCl Gasströmen jedoch zur Folge, dass sich in der durch die Kondensation abgeschiedenen organischen Komponenten signifikante Mengen an HCl und gegebenenfalls im Gasstrom vorhandenes Phosgen lösen. Das Einlösen dieser Komponenten verursacht nachfolgend signifikante Folgekosten zur Umwandlung von Phosgen und HCl mittels Natronlauge in Natriumchlorid und Natriumkarbonat. Gleichzeitig stellen die auf diesem Wege aus dem Prozess ausgeschleusten Phosgen- und HCl-Mengen einen nicht unerheblichen Chlorverlust dar, der insbesondere in einem Verfahren mit Chlorkreislauf unerwünscht ist.

Aufgabe der Erfindung ist es, ein verbessertes Trennverfahren bereitzustellen, das die Entfernung und gegebenenfalls die Wiederverwendung von organischen Komponenten aus einem HCl enthaltenden Rohgas ermöglicht.

Die vorliegende Erfindung hat insbesondere ferner die Verringerung des Verlustes von Wertstoffkomponenten wie Chlor in der Prozessgasreinigung von mit organischen Verbindungen verunreinigten Chlorwasserstoff enthaltenden Gasströmen zum Ziel.

Gegenstand der Erfindung ist ein Verfahren zur Entfernung organischer Komponenten aus einem gegebenenfalls heißen Chlorwasserstoff enthaltenden Rohgasstrom mit den Schritten:
A) Einstellung des zu reinigenden Rohgasstroms auf eine Temperatur von höchstens 40°C;
B) Kondensation mindestens eines Teils der organischen Komponenten des Rohgasstroms bei einer Temperatur von höchstens 0°C, bevorzugt von höchstens -10°C;
C) mindestens teilweise Adsorption der nach der Kondensation im vorgereinigten Gasstrom verbliebenen restlichen organischen Komponenten an einem Adsorptionsmedium;
D) gegebenenfalls nachfolgender Wärmeaustausch zwischen dem aus der Adsorption C) austretenden Gasstrom und dem in den Prozess eintretenden Rohgasstrom;
E) Bereitstellung des gereinigten Gasstromes;
dadurch gekennzeichnet, dass das unter B) erhaltene Kondensat einer Rektifikation F) unterzogen und der bei der Rektifikation anfallende Kopfgasstrom zusammen mit dem vorgereinigten Gasstrom des Adsorption nach Stufe C) zurückgeführt wird. Als Adsorptionsmittel für die Adsorption C) kommen hierbei in der Regel Aktivkohle, Zeolithe, Aluminiumoxid, Bentonit, Kieselgel oder auch metallorganische Komplexe zum Einsatz. Bevorzugt ist Aktivkohle. Gängige Apparatetypen zur Herstellung eines intensiven Gas-Adsorbens Kontaktes sind einfache Festbetten, Fließbetten, Wirbelbetten oder auch als ganzes bewegbare Festbetten.

Die Vorteile der adsorptiven Entfernung von Komponenten aus Gasströmen sind sehr hohe erreichbare Reinheiten des gereinigten Gasstromes sowie bei regenerativen Adsorptionsprozessen die Möglichkeit der Rückgewinnung der organischen Komponenten zur gezielten Entsorgung oder zur Rückführung in vorgängige Herstellungsverfahren.

Vorzugsweise handelt es sich daher bei einem solchen Chlorwasserstoff enthaltenden Rohgasstrom um einen chlorwasserstoffhaltigen Abstrom, der als Koppelprodukt beispielsweise in einem der folgenden Verfahren anfällt: der Isocyanatherstellung aus Phosgen und Aminen, der Säurechloridherstellung, der Polycarbonatherstellung, der Herstellung von Vinylchlorid aus Ethylendichlorid und der Chlorierung von Aromaten.

Die Temperatur des Rohgasstroms beträgt insbesondere bis zu 400°C, bevorzugt bis zu 250°C, besonders bevorzugt bis zu 150°C.

Bevorzugt ist ein Verfahren, das dadurch gekennzeichnet ist, dass die Abkühlung des Rohgasstroms zunächst in einem Kühler auf eine Temperatur von höchstens 45 °C erfolgt. Weiterhin bevorzugt erfolgt die Abkühlung des Rohgasstroms in einem zweiten Schritt, insbesondere in einem Rekuperator, auf eine Temperatur von höchstens 40 °C. In einer besonders bevorzugten Variante erfolgt der Wärmeaustausch zwischen dem aus der Adsorption austretenden Gasstrom und dem in den Prozess eintretenden Rohgasstrom in einem Rekuperator. Bevorzugt erfolgt die Abkühlung in einem ersten Schritt in einem Kühler auf eine Temperatur von höchstens 45 °C und in einem zweiten Schritt in einem Rekuperator auf eine Temperatur von höchstens 40 °C.

Eine besonders bevorzugte Variante des Verfahrens ist dadurch gekennzeichnet, dass das Adsorptionsmedium mit Hilfe eines weiteren aufgeheizten, insbesondere auf eine Temperatur von mindestens 50°C, geheizten Inertgasstromes regeneriert wird.

In einer bevorzugten Variante erfolgt die Rektifikation F) bei einer Temperatur im Sumpfverdampfer von mindestens 40°C, z. B. mindestens 60°C, bevorzugt mindestens 100°C. Das Verfahren wird besonders bevorzugt angewendet, wenn der zu reinigende Rohgasstrom im wesentlichen aus Chlorwasserstoff und ggf. bis zu 2 Gew.-% Phosgen besteht. Die aus dem Rohgasstrom abzutrennenden organischen Komponenten sind besonders bevorzugt im wesentlichen Kohlenwasserstoffe oder halogenierte Kohlenwasserstoffe, insbesondere bevorzugt aromatische Kohlenwasserstoffe wie Benzol, Toluol, Xylole und C₆-C₁₂-Aliphaten oder chlorierte Kohlenwasserstoffe wie Tetrachlorkohlenwasserstoff, Vinylchlorid und Dichlorethan oder chlorierte aromatische Kohlenwasserstoffe wie Hexachlorbenzol, Chlorbenzol oder Orthodichlorbenzol.

Eine weitere besonders bevorzugte Variante des Verfahrens ist dadurch gekennzeichnet, dass die Adsorption in wenigstens zwei Adsorptionsstufen erfolgt. Insbesondere bevorzugt wird dabei das Adsorptionsmedium der ersten Stufe des Schrittes C) mit Hilfe eines Teilstroms des Rohgasstroms regeneriert und der beladene Rohgasteilstrom gegebenenfalls mit dem in die Kondensation B) eintretenden Rohgasstrom vereinigt.

Eine bevorzugte Abwandlung des Verfahrens ist dadurch gekennzeichnet, dass das Adsorptionsmedium der ersten Stufe des Schrittes C) von Zeit zu Zeit im Wechsel mit dem Rohgasteilstrom mittels eines Inertgases im einfachen Durchgang regeneriert wird.

Das Verfahren wird besonders bevorzugt angewendet, wenn der chlorwasserstoffhaltige gereinigte Gasstrom in einem Produktionsverfahren zur Herstellung von Chlor aus Chlorwasserstoff und Sauerstoff weiterverwendet wird, insbesondere bei einer katalysierten Gasphasenoxidation von Chlorwasserstoff mit Sauerstoff oder einer nicht-thermischen Umsetzung von Chlorwasserstoff und Sauerstoff. Die Kopplung mit der katalysierten Gasphasenoxidation von Chlorwasserstoff mit Sauerstoff (Deacon-Verfahren) ist besonders bevorzugt.

Bevorzugt wird wie oben bereits beschrieben das als Deacon-Prozess bekannte katalytische Verfahren in Kombination mit dem erfindungsgemäßen Verfahren eingesetzt. Hierbei wird Chlorwasserstoff mit Sauerstoff in einer exothermen Gleichgewichtsreaktion zu Chlor oxidiert, wobei Wasserdampf anfällt. Die Reaktionstemperatur beträgt üblicherweise 150 bis 500°C, der übliche Reaktionsdruck beträgt 1 bis 25 bar. Da es sich um eine Gleichgewichtsreaktion handelt, ist es zweckmäßig, bei möglichst niedrigen Temperaturen zu arbeiten, bei denen der Katalysator noch eine ausreichende Aktivität aufweist. Ferner ist es zweckmäßig, Sauerstoff in überstöchiometrischen Mengen zum Chlorwasserstoff einzusetzen. Üblich ist beispielsweise ein zwei- bis vierfacher Sauerstoff-Überschuss. Da keine Selektivitätsverluste zu befürchten sind, kann es wirtschaftlich vorteilhaft sein, bei relativ hohem Druck und dementsprechend bei gegenüber Normaldruck längerer Verweilzeit zu arbeiten.

Geeignete bevorzugte Katalysatoren für das Deacon-Verfahren enthalten Rutheniumoxid, Rutheniumchlorid oder andere Rutheniumverbindungen auf Siliziumdioxid, Aluminiumoxid, Titandioxid oder Zirkondioxid als Träger. Geeignete Katalysatoren können beispielsweise durch Aufbringen von Rutheniumchlorid auf den Träger und anschließendes Trocknen oder Trocknen und Calcinieren erhalten werden. Geeignete Katalysatoren können ergänzend zu oder an Stelle einer Rutheniumverbindung auch Verbindungen anderer Edelmetalle, beispielsweise Gold, Palladium, Platin, Osmium, Iridium, Silber, Kupfer oder Rhenium enthalten. Geeignete Katalysatoren können ferner Chrom(III)oxid enthalten.

Die katalytische Chlorwasserstoff- Oxidation kann adiabatisch oder bevorzugt isotherm oder annähernd isotherm, diskontinuierlich, bevorzugt aber kontinuierlich als Fließ- oder Festbettverfahren, bevorzugt als Festbettverfahren, besonders bevorzugt in Rohrbündelreaktoren an Heterogenkatalysatoren bei einer Reaktortemperatur von 180 bis 500°C, bevorzugt 200 bis 400°C, besonders bevorzugt 220 bis 350°C und einem Druck von 1 bis 25 bar (1000 bis 25000 hPa), bevorzugt 1,2 bis 20 bar, besonders bevorzugt 1,5 bis 17 bar und insbesondere 2,0 bis 15 bar durchgeführt werden.

Übliche Reaktionsapparate, in denen die katalytische Chlorwasserstoff-Oxidation durchgeführt wird, sind Festbett- oder Wirbelbettreaktoren. Die katalytische Chlorwasserstoff- Oxidation kann bevorzugt auch mehrstufig durchgeführt werden.

Bei der adiabatischen, der isothermen oder annähernd isothermen Fahrweise können auch mehrere, also 2 bis 10, bevorzugt 2 bis 6, besonders bevorzugt 2 bis 5, insbesondere 2 bis 3, in Reihe geschaltete Reaktoren mit Zwischenkühlung eingesetzt werden. Der Chlorwasserstoff kann entweder vollständig zusammen mit dem Sauerstoff vor dem ersten Reaktor oder über die verschiedenen Reaktoren verteilt zugegeben werden. Diese Reihenschaltung einzelner Reaktoren kann auch in einem Apparat zusammengeführt werden.

Eine weitere bevorzugte Ausführungsform einer für das Verfahren geeigneten Vorrichtung besteht darin, dass man eine strukturierte Katalysatorschüttung einsetzt, bei der die Katalysatoraktivität in Strömungsrichtung ansteigt. Eine solche Strukturierung der Katalysatorschüttung kann durch unterschiedliche Tränkung der Katalysatorträger mit Aktivmasse oder durch unterschiedliche Verdünnung des Katalysators mit einem Inertmaterial erfolgen. Als Inertmaterial können beispielsweise Ringe, Zylinder oder Kugeln aus Titandioxid, Zirkondioxid oder deren Gemischen, Aluminiumoxid, Steatit, Keramik, Glas, Graphit, Edelstahl oder Nickellegierungen eingesetzt werden. Beim bevorzugten Einsatz von Katalysatorformkörpern sollte das Inertmaterial bevorzugt ähnliche äußeren Abmessungen haben.

Als Katalysatorformkörper eignen sich Formkörper mit beliebigen Formen, bevorzugt sind Tabletten, Ringe, Zylinder, Sterne, Wagenräder oder Kugeln, besonders bevorzugt sind Ringe, Zylinder oder Sternstränge als Form.

Als Heterogenkatalysatoren eignen sich insbesondere Rutheniumverbindungen oder Kupferverbindungen auf Trägermaterialen, die auch dotiert sein können, bevorzugt sind gegebenenfalls dotierte Rutheniumkatalysatoren. Als Trägermaterialen eignen sich beispielsweise Siliziumdioxid, Graphit, Titandioxid mit Rutil- oder Anatas-Struktur, Zirkondioxid, Aluminiumoxid oder deren Gemische, bevorzugt Titandioxid, Zirkondioxid, Aluminiumoxid oder deren Gemische, besonders bevorzugt γ- oder δ-Aluminiumoxid oder deren Gemische.

Die Kupfer- bzw. die Rutheniumträgerkatalysatoren können beispielsweise durch Tränkung des Trägermaterials mit wässrigen Lösungen von CuCl₂ bzw. RuCl₃ und gegebenenfalls eines Promotors zur Dotierung, bevorzugt in Form ihrer Chloride, erhalten werden. Die Formgebung des Katalysators kann nach oder bevorzugt vor der Tränkung des Trägermaterials erfolgen.

Zur Dotierung der Katalysatoren eignen sich als Promotoren Alkalimetalle wie Lithium, Natrium, Kalium, Rubidium und Cäsium, bevorzugt Lithium, Natrium und Kalium, besonders bevorzugt Kalium, Erdalkalimetalle wie Magnesium, Calcium, Strontium und Barium, bevorzugt Magnesium und Calcium, besonders bevorzugt Magnesium, Seltenerdmetalle wie Scandium, Yttrium, Lanthan, Cer, Praseodym und Neodym, bevorzugt Scandium, Yttrium, Lanthan und Cer, besonders bevorzugt Lanthan und Cer, oder deren Gemische.

Die Formkörper können anschließend bei einer Temperatur von 100 bis 400°C, bevorzugt 100 bis 300°C beispielsweise unter einer Stickstoff-, Argon- oder Luftatmosphäre getrocknet und gegebenenfalls kalziniert werden. Bevorzugt werden die Formkörper zunächst bei 100 bis 150°C getrocknet und anschließend bei 200 bis 400°C kalziniert.

Der Umsatz an Chlorwasserstoff im einfachen Durchgang kann bevorzugt auf 15 bis 90 %, bevorzugt 40 bis 85%, besonders bevorzugt 50 bis 70% begrenzt werden. Nicht umgesetzter Chlorwasserstoff kann nach Abtrennung teilweise oder vollständig in die katalytische Chlorwasserstoff-Oxidation zurückgeführt werden. Das Volumenverhältnis von Chlorwasserstoff zu Sauerstoff am Reaktoreintritt beträgt bevorzugt 1:1 bis 20:1, bevorzugt 1:1 bis 8:1, besonders bevorzugt 1:1 bis 5:1.

Die Reaktionswärme der katalytischen Chlorwasserstoff-Oxidation kann in vorteilhafter Weise zur Erzeugung von Hochdruck-Wasserdampf genutzt werden. Dieser kann zum Betrieb eines Phosgenierungsreaktors und oder von Destillationskolonnen, insbesondere von Isocyanat-Destillationskolonnen genutzt werden.

In einem weiteren Schritt wird das gebildete Chlor abgetrennt. Der Abtrennschritt umfasst üblicherweise mehrere Stufen, nämlich die Abtrennung und gegebenenfalls Rückführung von nicht umgesetztem Chlorwasserstoff aus dem Produktgasstrom der katalytischen ChlorwasserstoffOxidation, die Trocknung des erhaltenen, im wesentlichen Chlor und Sauerstoff enthaltenden Stroms sowie die Abtrennung von Chlor aus dem getrockneten Strom.

Die Abtrennung von nicht umgesetztem Chlorwasserstoff und von gebildetem Wasserdampf kann durch Auskondensieren von wässriger Salzsäure aus dem Produktgasstrom der Chlorwasserstoffoxidation durch Abkühlung erfolgen. Chlorwasserstoff kann auch in verdünnter Salzsäure oder Wasser absorbiert werden.

Das Verfahren wird nachstehend anhand der Figuren beispielhaft erläutert. Es zeigt:

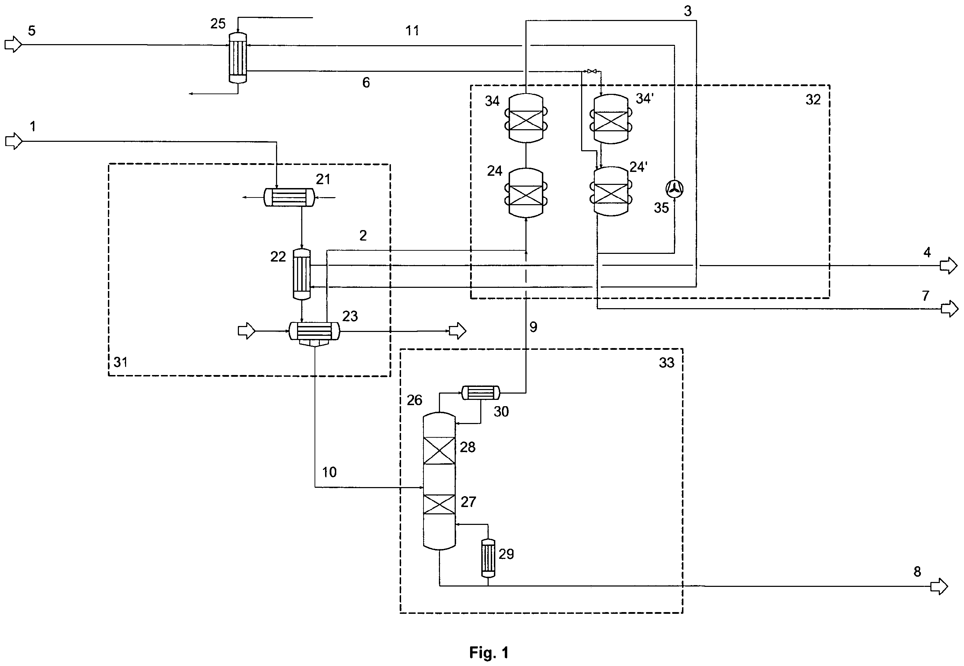

- Fig. 1: ein Verfahrensfließbild der erfindungsgemäßen Rohgasreinigung

### Beispiele

### Beispiel 1

Das Rohgas 1 (hier Chlorwasserstoffgas aus einer TDI-Produktion) wird in die erste Stufe 31 des Verfahrens geleitet. Dort wird es in einem Kühler 21 vorgekühlt und durch den Rekuperator 22 zur Einstellung einer Temperatur unterhalb von 10°C geleitet. Organische Verunreinigungen wie Hexachlorbenzol, Orthodichlorbenzol oder Chlorbenzol werden im Kondensator 23 bei einer Temperatur von -35°C kondensiert und als Strom 10 abgeführt (Fig. 1). Gleichzeitig werden wie bereits oben beschrieben HCl und ggf. Phosgen in das Kondensat eingelöst.

Das vorgereinigte Rohgas 2 gelangt in die zweite Stufe 32 des Verfahrens und wird über ein erstes Adsorberbett 24 geleitet, in dem organische Verunreinigungen weiter abgereichert werden.

Das beladene Adsorberbett 24', das im Wechsel mit dem Adsorberbett 24 betrieben wird, wird mit einem Inertgas 6 gereinigt, das aus frischem Inertgas 5 und einem Rückstrom 11 zusammengesetzt ist und im Wärmetauscher 25 vorerhitzt wird. Der Rückstrom 11 wird mit einem Gebläse 35 gefördert.

Nach Durchlaufen des Adsorbers 24 wird der beladene Regenerationsgasstrom 7 aufgearbeitet.

Anschließend wird der Gasstrom aus dem ersten Adsorberbett 24 auf ein redundantes Adsorbersystem 34, 34' aus Aktivkohle geleitet. In diesem redundanten Adsorbersystem steht immer jeweils ein Adsorber 34 zur Adsorption zur Verfügung, während der redundante Adsorber 34' regeneriert werden kann. Die Regeneration kann dabei wahlweise mit heißem Inertgas oder (jeweils in Fig.1 nicht dargestellt) mit heißem Rohgas oder mit Inertgas im Kreislaufstrom erfolgen.

Der adsorptiv gereinigte Gasstrom 3 verlässt die zweite Stufe des Verfahrens und wird nach Durchlaufen des Rekuperators 22 unter Wärmeaustausch mit dem Rohgasstrom 1 aus der ersten Stufe 31 des Verfahrens dem der Gasreinigung nachfolgenden Deacon-Verfahren (nicht gezeichnet) als Strom 4 bereitgestellt und dort zu Chlor oxidiert. Stufe 31 und Stufe 32 des Verfahrens werden bis auf die Apparate- und Leitungsdruckverluste isobar bei einem Druck von 6 bar betrieben.

Das in der ersten Stufe 31 des Verfahrens anfallende Kondensat 10 wird der dritten Verfahrensstufe 33 (Rektifikation) zugeleitet. In der in Fig. 1 dargestellten Ausführungsform besteht die dritte Stufe 33 des Verfahrens im wesentlichen aus einer Rektifikationskolonne 26, mit einem Verstärkungsteil 28, einem Abtriebsteil 27 sowie einem Sumpfverdampfer 29 und einem Kopfkondensator 30. Der unter anderem mit HCl und Phosgen beladene Organikstrom aus Stufe 31 wird der Rektifikationskolonne 26 zwischen dem Abtriebs- 27 und dem Verstärkungsteil 28 zugeleitet. Im Abtriebsteil 27 steigt der im Sumpfverdampfer 29 generierte Dampf im Gegenstrom zu dem die Kolonne 26 herabfließenden Organikstrom nach oben. Hierbei wird der HCl- und Phosgenanteil im aufsteigenden Dampfstrom kontinuierlich erhöht. Der herablaufenden Flüssigkeit wird gleichzeitig HCl und Phosgen entzogen. Im Verstärkungsteil 28 der Kolonne kommt es zu einer weiteren Aufkonzentrierung des Gasstromes mit HCl und Phosgen während die organischen Komponenten abgereichert werden. Die HCl/Phosgenkonzentration bzw. die Organikkonzentration am Kolonnenkopf bzw. hinter dem Kopfkondensator 30 der Kolonne wird im wesentlichen durch die Kondensatortemperatur und den damit erzeugten Kolonnenrücklauf beeinflusst. Der Kopfkondensator 30 ist als Dephlegmator ausgebildet, so dass ein gasförmiger, vor allem Phosgen und HCl enthaltender Brüdenstrom 9 über Kopf der Rektifikation entnommen wird und zusammen mit Strom 2 auf die Adsorption der Stufe 32 des Verfahrens geleitet wird. Die Kondensationstemperatur der Rektifikation ist so zu wählen, dass die Organikfracht in Strom 9 nur ein Bruchteil der in Strom 1 vorliegenden Organikfracht beträgt. Die Kondensatortemperatur beträgt -10°C.

Der am Kolonnensumpf abgezogene Organikstrom 8 ist weitestgehend frei von HCl und Phosgen und wird zur weiteren Verarbeitung bzw. Entsorgung bereitgestellt. Die Sumpftemperatur beträgt 140°C. Der Kolonnendruck liegt oberhalb des Druckniveaus der Stufe 32 des Verfahrens. Damit kann Strom 9 ohne eine Kompression in die Adsorption der zweiten Verfahrensstufe eingeleitet werden.

## Patentansprüche

1. Verfahren zur Entfernung organischer Komponenten aus einem Chlorwasserstoff enthaltenden Rohgasstrom mit den Schritten:
A) Einstellung des zu reinigenden Rohgasstroms (1) auf eine Temperatur von höchstens 40°C;
B) Kondensation mindestens eines Teils der organischen Komponenten des Rohgasstroms bei einer Temperatur von höchstens 0°C, bevorzugt von höchstens-10°C;
C) mindestens teilweise Adsorption der nach der Kondensation im vorgereinigten Gasstrom verbliebenen restlichen organischen Komponenten an einem ersten Adsorptionsmedium;
D) gegebenenfalls nachfolgender Wärmeaustausch zwischen dem aus der Adsorption C) austretenden Gasstrom und dem in den Prozess eintretenden Rohgasstrom;
E) Bereitstellung des gereinigten Gasstromes (4);
**dadurch gekennzeichnet, dass** das unter B) erhaltene Kondensat einer Rektifikation F) unterzogen und der bei der Rektifikation anfallende Kopfgasstrom (9) zusammen mit dem vorgereinigten Gasstrom der Adsorption nach Stufe C) zugeführt wird.

2. Verfahren nach Anspruch 1, **dadurch gekennzeichnet, dass** das gesamte Verfahren bei einem Druck von bis zu 20 bar, bevorzugt bei 2 bis 12 bar erfolgt.

3. Verfahren nach Anspruch 1 oder 2, **dadurch gekennzeichnet, dass** die Rektifikation bei einer Temperatur im Sumpfverdampfer (29) von mindestens 40°C, bevorzugt mindestens 100°C erfolgt.

4. Verfahren nach einem der Ansprüche 1 bis 3, **dadurch gekennzeichnet, dass** der Wärmeaustausch zwischen dem aus der Adsorption 32 austretenden Gasstrom 3 und dem in den Prozess eintretenden Rohgasstrom 1 in einem Rekuperator 22 erfolgt.

5. Verfahren nach einem der Ansprüche 1 bis 4, **dadurch gekennzeichnet, dass** die Temperatureinstellung A) in einem ersten Schritt in einem Kühler (21) auf eine Temperatur von höchstens 45°C und in einem zweiten Schritt in einem Rekuperator auf eine Temperatur von höchstens 40°C erfolgt.

6. Verfahren nach einem der Ansprüche 1 bis 5, **dadurch gekennzeichnet, dass** das Adsorptionsmedium des Adsorbers (24, 24') der Adsorptionsstufe (32) mit Hilfe eines aufgeheizten Inertgasstromes regeneriert wird.

7. Verfahren nach einem der Ansprüche 1 bis 6, **dadurch gekennzeichnet, dass** der zu reinigende Rohgasstrom (1) im Wesentlichen aus Chlorwasserstoff und ggf. bis zu 2 Gew.-% Phosgen besteht.

8. Verfahren nach einem der Ansprüche 1 bis 7, **dadurch gekennzeichnet, dass** die aus dem Rohgasstrom 1 abzutrennenden organischen Komponenten im wesentlichen Kohlenwasserstoffe oder halogenierte Kohlenwasserstoffe, insbesondere aromatische Kohlenwasserstoffe wie Benzol, Toluol, Xylole und C₆-C₁₂-Aliphaten oder chlorierte Kohlenwasserstoffe wie Tetrachlorkohlenstoff, Vinylchlorid und Dichlorethan oder chlorierte aromatische Kohlenwasserstoffe wie Hexachlorbenzol, Chlorbenzol oder Orthodichlorbenzol sind.

9. Verfahren nach einem der Ansprüche 1 bis 8, **dadurch gekennzeichnet, dass** die Adsorption (32) in wenigstens zwei Adsorptionsstufen erfolgt.

10. Verfahren nach einem der Ansprüche 1 bis 9, **dadurch gekennzeichnet, dass** der Rohgasstrom 1 im Wesentlichen aus Chlorwasserstoff und ggf. Phosgen besteht und der gereinigte Chlorwasserstoff enthaltende Gasstrom in einem Produktionsverfahren zur Herstellung von Chlor aus Chlorwasserstoff und Sauerstoff weiterverwendet wird, insbesondere bei einer katalysierten Gasphasenoxidation von Chlorwasserstoff mit Sauerstoff oder einer nicht-thermischen Umsetzung von Chlorwasserstoff und Sauerstoff.

11. Verfahren nach einem der Ansprüche 1 bis 10, **dadurch gekennzeichnet, dass** der Rohgasstrom 1 ein Chlorwasserstoff enthaltender Abstrom ist, der als Koppelprodukt beispielsweise in einem der folgenden Verfahren anfällt: der Isocyanatherstellung aus Phosgen und Aminen, der Säurechloridherstellung, der Polycarbonatherstellung, der Herstellung von Vinylchlorid aus Ethylendichlorid und der Chlorierung von Aromaten.

## Claims

1. Process for removing organic components from a hydrogen-chloride-containing crude gas stream, comprising the steps:
A) adjustment of the crude gas stream (1) that is to be purified to a temperature not exceeding 40°C;
B) condensation of at least some of the organic components of the crude gas stream at a temperature not exceeding 0°C, preferably not exceeding -10°C;
C) at least partial adsorption of the residual organic components that remain in the prepurified gas stream after the condensation, on a first adsorption medium;
D) optional subsequent heat exchange between the gas stream leaving the adsorption C) and the crude gas stream entering the process;
E) provision of the purified gas stream (4);
**characterised in that** the condensate obtained under B) is subjected to a rectification F) and the head gas stream (9) obtained in the rectification is fed to the adsorption according to stage C) together with the prepurified gas stream.

2. Process according to claim 1, **characterised in that** the whole process takes place at a pressure of up to 20 bar, preferably at from 2 to 12 bar.

3. Process according to claim 1 or 2, **characterised in that** the rectification takes place at a temperature in the sump evaporator (29) of at least 40°C, preferably at least 100°C.

4. Process according to any one of claims 1 to 3, **characterised in that** the heat exchange between the gas stream 3 leaving the adsorption 32 and the crude gas stream 1 entering the process takes place in a recuperator 22.

5. Process according to any one of claims 1 to 4, **characterised in that** the temperature adjustment A) takes place in a first step in a cooler (21) to a temperature not exceeding 45°C and in a second step in a recuperator to a temperature not exceeding 40°C.

6. Process according to any one of claims 1 to 5, **characterised in that** the adsorption medium of the adsorber (24, 24') of the adsorption stage (32) is regenerated with the aid of a heated inert gas stream.

7. Process according to any one of claims 1 to 6, **characterised in that** the crude gas stream (1) that is to be purified consists substantially of hydrogen chloride and optionally up to 2 wt.% phosgene.

8. Process according to any one of claims 1 to 7, **characterised in that** the organic components that are to be separated from the crude gas stream 1 are substantially hydrocarbons or halogenated hydrocarbons, in particular aromatic hydrocarbons such as benzene, toluene, xylenes and C₆-C₁₂-aliphatic compounds, or chlorinated hydrocarbons such as carbon tetrachloride, vinyl chloride and dichloroethane, or chlorinated aromatic hydrocarbons such as hexachlorobenzene, chlorobenzene or orthodichlorobenzene.

9. Process according to any one of claims 1 to 8, **characterised in that** the adsorption (32) takes place in at least two adsorption stages.

10. Process according to any one of claims 1 to 9, **characterised in that** the crude gas stream 1 consists substantially of hydrogen chloride and optionally phosgene, and the purified hydrogen-chloride-containing gas stream is used further in a production process for the preparation of chlorine from hydrogen chloride and oxygen, in particular in a catalysed gas-phase oxidation of hydrogen chloride with oxygen or in a non-thermal reaction of hydrogen chloride and oxygen.

11. Process according to any one of claims 1 to 10, **characterised in that** the crude gas stream 1 is a hydrogen-chloride-containing waste stream obtained as by-product in one of the following processes, for example: isocyanate preparation from phosgene and amines, acid chloride preparation, polycarbonate preparation, the preparation of vinyl chloride from ethylene dichloride, and the chlorination of aromatic compounds.

## Revendications

1. Procédé d'élimination de composants organiques d'un courant gazeux brut contenant du chlorure d'hydrogène, comprenant les étapes suivantes :
A) l'ajustement du courant gazeux brut à purifier (1) à une température d'au plus 40 °C ;
B) la condensation d'au moins une partie des composants organiques du courant gazeux brut à une température d'au plus 0 °C, de préférence d'au plus-10 °C ;
C) l'adsorption au moins partielle des composants organiques résiduels restant dans le courant gazeux pré-purifié après la condensation dans un premier milieu d'adsorption ;
D) éventuellement l'échange de chaleur ultérieur entre le courant gazeux sortant de l'adsorption C) et le courant gazeux brut entrant dans le procédé ;
E) la mise à disposition du courant gazeux purifié (4) ;
**caractérisé en ce que** le condensat obtenu en B) est soumis à une rectification F) et le courant gazeux de tête (9) formé lors de la rectification est introduit conjointement avec le courant gazeux pré-purifié dans l'adsorption selon l'étape C).

2. Procédé selon la revendication 1, **caractérisé en ce que** l'ensemble du procédé a lieu à une pression de jusqu'à 20 bar, de préférence de 2 à 12 bar.

3. Procédé selon la revendication 1 ou 2, **caractérisé en ce que** la rectification a lieu à une température dans l'évaporateur de fond (29) d'au moins 40 °C, de préférence d'au moins 100 °C.

4. Procédé selon l'une quelconque des revendications 1 à 3, **caractérisé en ce que** l'échange de chaleur entre le courant gazeux 3 sortant de l'adsorption 32 et le courant gazeux brut 1 entrant dans le procédé a lieu dans un récupérateur 22.

5. Procédé selon l'une quelconque des revendications 1 à 4, **caractérisé en ce que** l'ajustement de température A) a lieu lors d'une première étape dans un refroidisseur (21) à une température d'au plus 45 °C et lors d'une seconde étape dans un récupérateur à une température d'au plus 40 °C.

6. Procédé selon l'une quelconque des revendications 1 à 5, **caractérisé en ce que** le milieu d'adsorption de l'adsorbeur (24, 24') de l'étape d'adsorption (32) est régénéré à l'aide d'un courant gazeux inerte chauffé.

7. Procédé selon l'une quelconque des revendications 1 à 6, **caractérisé en ce que** le courant gazeux brut à purifier (1) est essentiellement constitué de chlorure d'hydrogène et éventuellement de jusqu'à 2 % en poids de phosgène.

8. Procédé selon l'une quelconque des revendications 1 à 7, **caractérisé en ce que** les composants organiques à séparer du courant gazeux brut 1 sont essentiellement des hydrocarbures ou des hydrocarbures halogénés, notamment des hydrocarbures aromatiques tels que le benzène, le toluène, des xylènes et des composés aliphatiques en C₆-C₁₂, ou des hydrocarbures chlorés tels que le tétrachlorocarbone, le chlorure de vinyle et le dichloroéthane, ou des hydrocarbures aromatiques chlorés tels que l'hexachlorobenzène, le chlorobenzène ou l'orthodichlorobenzène.

9. Procédé selon l'une quelconque des revendications 1 à 8, **caractérisé en ce que** l'adsorption (32) a lieu dans en au moins deux étapes d'adsorption.

10. Procédé selon l'une quelconque des revendications 1 à 9, **caractérisé en ce que** le courant gazeux brut 1 est essentiellement constitué de chlorure d'hydrogène et éventuellement de phosgène, et le courant gazeux contenant du chlorure d'hydrogène purifié est utilisé dans un procédé de production pour la fabrication de chlore à partir de chlorure d'hydrogène et d'oxygène, notamment dans une oxydation catalysée en phase gazeuse de chlorure d'hydrogène avec de l'oxygène ou une réaction non thermique de chlorure d'hydrogène et d'oxygène.

11. Procédé selon l'une quelconque des revendications 1 à 10, **caractérisé en ce que** le courant gazeux brut 1 est un courant résiduaire contenant du chlorure d'hydrogène, qui se forme en tant que produit secondaire par exemple dans un des procédés suivants : la fabrication d'isocyanates à partir de phosgène et d'amines, la fabrication de chlorures d'acides, la fabrication de polycarbonates, la fabrication de chlorure de vinyle à partir de dichlorure d'éthylène et la chloration de composés aromatiques.
